(19) Europäisches Patentamt
European Patent Office
Office européen des brevets

(11) EP 0 783 294 B1

(12) EUROPEAN PATENT SPECIFICATION

(45) Date of publication and mention of the grant of the patent:
**10.02.1999 Bulletin 1999/06**

(21) Application number: **95937677.3**

(22) Date of filing: **30.10.1995**

(51) Int. Cl.$^6$: **A61K 7/48**

(86) International application number:
**PCT/US95/14045**

(87) International publication number:
**WO 96/14054 (17.05.1996 Gazette 1996/22)**

# (54) TOPICAL AQUEOUS SKIN CARE COMPOSITION WITH HYDROGEL THICKENER AND WATER INSOLUBLE SILICONE CONDITIONING AGENT

TOPISCHES WÄSSRIGES HAUTPFLEGEMITTEL ENTHALTEND EIN HYDROGEL-EINDICKER UND EIN WASSERUNLÖSLICHE SILIKONKONDITIONIERUNGSAGENS

COMPOSITION TOPIQUE AQUEUSE POUR SOIN DE LA PEAU CONTENANT UN HYDROGEL EPAISSISSEUR ET UN AGENT TRAITANT AU SILICONE INSOLUBLE DANS L'EAU

(84) Designated Contracting States:
**AT BE CH DE DK ES FR GB GR IE IT LI LU NL PT SE**

(30) Priority: **07.11.1994 US 335274**

(43) Date of publication of application:
**16.07.1997 Bulletin 1997/29**

(73) Proprietor:
**THE PROCTER & GAMBLE COMPANY**
**Cincinnati, Ohio 45202 (US)**

(72) Inventors:
- **SIMMONS, Mason Stanley**
**West Chester, OH 45069 (US)**
- **WILSON, Bryce William**
**Hamilton, OH 45011 (US)**

(74) Representative:
**Woof, Victoria et al**
**Patent Dept.,**
**Procter & Gamble Technical Centres Limited,**
**Rusham Park,**
**Whitehall Lane**
**Egham TW20 9NW (GB)**

(56) References cited:
**EP-A- 0 392 426**
**EP-A- 0 477 053**
**WO-A-94/08555**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Xerox (UK) Business Services
2.16.7/3.6

## Description

FIELD OF INVENTION

This invention relates to topical skin care compositions. More particularly, this invention relates to topical aqueous skin care compositions with hydrogel thickening agent and water-insoluble silicone conditioning agent.

BACKGROUND OF THE INVENTION

A wide variety of skin care compositions are topically applied for conditioning the skin. Exemplary types of such compositions include hand and facial conditioning compositions, sunscreen compositions, after-shave compositions, and skin renewal compositions.

It is desirable for these products to be thickened, such as in the form of a gel, so that the product is less thin and runny, is easy to apply to the skin, and is suitable for suspending other active ingredients, e.g. skin conditioners. A wide variety of thickeners have been used in the area of topical skin care compositions. These include, for example, hydroxylated vinylic polymers, homopolymers of acrylic acid crosslinked with an alkyl ether of pentaerythritol or an ally ether of sucrose, carboxymethylcelluloses such as hydroxypropyl methylcellulose, microcrystalline cellulose, aluminum silicates and magnesium aluminum silicates, ethylene glycol di- and mono- stearates, etc. It is particularly common to use acrylic acid crosslinked polymers. These polymers, when present in skin care compositions, are often referred to in the art as "carbomer" polymers, according to Cosmetic, Toiletries, and Cosmetics Association (CTFA) nomenclature. Acrylic acid crosslinked polymer ("carbomers") form gels which rapidly breakdown upon contact with skin surface electrolytes, thereby transforming from a gel to an easily spreadable liquid, for even distribution across the skin surface. However these carbomers are, unfortunately, also characterized by a relatively sticky or tacky feel.

It is also desirable to incorporate silicone emollients as conditioning agents, into topical skin care compositions for short term skin feel benefits (e.g., volatile silicones such as cyclomethicone) or long term emolliency (e.g., nonvolatile silicones such as dimethicone).

Unfortunately, it can be difficult to incorporate silicone emollients into thickened, aqueous topical skin care compositions without causing the product to become highly opaque. In order to formulate such products so that they are clear or translucent, it has been disclosed to incorporate surfactants, especially nonionic surfactants to act as emulsifers. Prior efforts to obtain clear or translucent thickened conditioning compositions containing silicone emollients have several drawbacks. First, the use of surfactants can undesirably increase the incidence of skin irritation across a portion of the public. Second, the use of surfactants can also result in a more sticky or tacky feel on the skin. Third, the use of surfactants adds cost to the product. Fourth, as discussed above, the carbomer thickeners most often used in these types of compositions can also impart a tacky skin feel, thereby exacerbating the skin feel characteristics adverse of the surfactant. It has also been disclosed (EP-A-0 392 426) that a powdered carrier material of a cross-linked hydrophobic copolymer can be used to maintain clarity without the use of surfactants However, this does not address the problem of tackiness and requires an additional ingredient.

It is an object of this invention to provide thickened, aqueous skin care compositions containing water-insoluble silicone emollients which remain translucent and which have improved skin feel and exhibit reduced tackiness.

It is another object of this invention to provide a product as described above which further exhibits excellent skin sensitivity.

These and other benefits as may be discussed herein or apparent to one skilled in the art can be provided according to the invention which is described below.

All percentages herein are by weight of the composition unless otherwise indicated. All ratios are weight ratios unless otherwise indicated. All percentages, ratios, and levels of ingredients referred to herein are based on the actual amount of the ingredient, and do not include solvents, fillers, or other materials with which the ingredient may be combined as commercially available products, unless otherwise indicated.

The invention hereof can comprise, consist of, or consist essentially of the essential elements described herein as well as any of the preferred or optional ingredients also described herein. Nothing herein is meant to be interpreted to exclude the use or presence of other components or steps except as may be otherwise indicated.

SUMMARY OF THE INVENTION

The present invention provides a thickened, aqueous composition suitable for topical application to the skin. The present compositions comprise water, water-insoluble silicone emollient, and a highly absorbent crosslinked, hydrogel forming polymeric gelling agent. The particular viscosity of the compositions of the present invention is not critical, although for purposes hereof the compositions should be sufficiently thickened such that they have a viscosity of at least about 4,000 centipoise at 25°C. The silicone emollient is suspended by hydrogel, and is in the form of droplets

having a number average particle size of at least about 5 microns. The compositions hereof are substantially free of surfactants, but nevertheless remain translucent. Furthermore, the compositions have low skin irritation, excellent skin feel, and low tackiness.

More specifically, the present invention provides a translucent, hydrogel, topical skin care composition comprising:

(a) from 0.1% to 10%, by weight, hydrogel forming polymeric gelling agent, calculated on dry polymeric gelling agent weight basis, wherein said gelling agent is a crosslinked polymer having an absorptive capacity of at least about 40 grammes of water per gramme of gelling agent and
not having a Tg below 140°C; (b) from 0.1% to 15%, by weight, water insoluble silicone emollient; said emollient being in the form of droplets having a number average particle size of from 5 microns to 4,000 microns;
(c) from 50% to 99.8%, by weight, water;

wherein said composition has a viscosity at 25°C of at least about 4,000 centipoise and is substantially free of surfactants.

The present invention, as well as various optional and preferred embodiments thereof, are described in more detail below.

DETAILED DESCRIPTION OF THE INVENTION

Hydrogel Forming Polymeric Gelling Agent

The compositions will comprise aqueous gel, i.e. "hydrogel", formed from a hydrogel forming polymeric gelling agent and water. The compositions will contain from 0.1% to 10%, by weight of the composition, preferably from 0.2% to 5%, more preferably from 0.3% to 1%, of such hydrogel forming polymeric gelling agent, calculated based on the dry weight of the hydrogel forming polymeric gelling agent.

The hydrogel forming polymeric gelling agent hereof is highly absorbent of water, and will be able to absorb at least about 40 g water (deionized) per gram of gelling agent, preferably at least about 60 g/g, more preferably at least about 80 g/g. These values, referred to as "Absorptive Capacity" herein can be determined according to the procedure in the Absorptive Capacity "Tea Bag" test in the Experimental Section below.

The hydrogel forming polymeric gelling agent hereof will also preferably be characterized by an extractable polymer content of no more than about 20%, more preferably no more than about 12%, most preferably no more than about 10%. The extractable polymer content is determined according to the procedures set forth in the Experimental Section, below.

The hydrogel forming polymeric material, when in dry form, will generally be in the form of particles or fibers. Particles will preferably have a weight average particle size (diameter, or equivalent diameter in the case of non-spherical particles) of from 5 to 500 microns, preferably from 10 to 60 microns, more preferably from 10 to 50 microns. Fibrous hydrogel forming polymeric gelling agent preferably have diameters (or equivalent diameters in the case of non-round fibers) of from 5 to 100 microns, preferably from

15 to 50 microns, and lengths of from 0.1 mm to 5 mm more preferably from 0.5 mm to 2 mm. It is contemplated that larger or smaller particles or fibers can be used, although they are not preferred, as larger particles or fibers may provide a grainier or a more string-like product feel, and smaller particles may result in processing difficulties. If relatively large particles or fibers are used during manufacture, improved finished product aesthetics can be obtained by sheer mixing the compositions or other mixing operations during processing of the compositions.

In general, the hydrogel forming polymeric gelling agent materials of the present invention are at least partially crosslinked polymers prepared from polymerizable, unsaturated acid-containing monomers which are water-soluble or become water-soluble upon hydrolysis. These include monoethylenically unsaturated compounds having at least one hydrophilic radical, including olefinically unsaturated acids and anhydrides which contain at least one carbon-carbon olefinic double bond.

With respect to these monomers, water-soluble means that the monomer is soluble in deionized water at 25°C at a level of at least 0.2%, preferably at least 1.0%.

Upon polymerization, monomeric units as described above will constitute from about 25 mole percent to 99.99 mole percent, more preferably from about 50 mole percent to 99.99 mole percent, most preferably at least about 75 mole percent of the polymeric gelling agent material (dry polymer weight basis). Two or more different monomer types of the previously described acid group-containing monomers may be copolymerized in order to provide the hydrogel-forming polymeric gelling material. Exemplary types of such acid groups and other hydrophilic groups include carboxyl, carboxylic acid anhydride, carboxylic salt, sulfonic acid, sulfonic acid salt, hydroxyl, ether, amide, amino and ammonium salt groups.

Hydrogel forming polymeric gelling agents suitable for use herein are well known in the art, and are described, for example, in U.S. Patent 4,076,663, Masuda et al., issued February 28, 1978; U.S. Patent 4,062,817, Westerman, issued December 13, 1977; U.S. Patent 4,286,082, Tsubakimoto et al., issued August 25, 1981; U.S. Patent 5,061,259, Goldman et al., issued October 29, 1991, and U.S. Patent 4,654,039, Brandt et al., issued March 31, 1987.

Hydrogel forming polymeric gelling agents suitable for use herein are also described in U.S. Patent 4,731,067, Le-Khac, issued March 15, 1988, U.S. Patent 4,743,244, Le-Khac, issued May 10, 1988, U.S. Patent 4,813,945, Le-Khac, issued March 21, 1989, U.S. Patent 4,880,868, Le-Khac, issued November 14, 1989, U.S. Patent 4,892,533, Le-Khac, issued January 9, 1990, U.S. Patent 5,026,784, Le-Khac, issued June 25, 1991, U.S. Patent 5,079,306, Le-Khac, issued January 7, 1992, U.S. Patent 5,151,465, Le-Khac, issued September 29, 1992, U.S. Patent 4,861,539, Allen, Farrer, and Flesher, issued August 29, 1989, and U.S. Patent 4,962,172, Allen, Fatter, and Flesher, issued October 9, 1990.

Examples of suitable water-soluble monomers from which monomer units of the polymers hereof can be derived are as follows:

1. Carboxyl group-containing monomers (carboxylic acid-containing): monoethylenically unsaturated mono or poly-carboxylic acids, such as (meth) acrylic acid (meaning acrylic acid or methacrylic acid; similar notations are used hereinafter), maleic acid, fumaric acid, sorbic acid, itaconic acid, citraconic acid, tricarboxy ethylene, and ethacrylic acid;
2. Carboxylic acid anhydride group-containing monomers: monoethylenically unsaturated polycarboxylic acid anhydrides, such as maleic anhydride;
3. Carboxylic acid salt-containing monomers: water-soluble salts (alkali metal salts, ammonium salts, amine salts, etc.) of monoethylenically unsaturated mono- or poly- carboxylic acids [such as sodium (meth)acrylate, trimethylamine (meth)acrylate, triethanolamine (meth) acrylate, sodium maleate, methylamine maleate];
4. Sulfonic acid group-containing monomers: aliphatic or aromatic vinyl sulfonic acids (such as vinylsulfonic acid, ally sulfonic acid, vinyltoluenesulfonic acid, styrene sulfonic acid), (meth)acrylic sulfonic acids [such as sulfopropyl (meth) acrylate, 2-hydroxy-3-(meth)acryloxy propyl sulfonic acid, 2-acylamido-2-methyl propane sulfonic acid];
5. Sulfonic acid salt group-containing monomers: alkali metal salts, ammonium salts, amine salts of sulfonic acid group-containing monomers as mentioned above.
6. Hydroxyl group-containing monomers: monoethylenically unsaturated alcohols [such as (meth)allyl alcohol], monoethylenically unsaturated ethers or esters of polyols (alkylene glycols, glycerol, polyoxyalkylene polyols), such as hydroxethyl (meth)acrylate, hydroxypropyl (meth)acrylate, triethylene glycol (meth)acrylate, poly(oxyethylene oxypropylene) glycol mono (meth)allyl ether (in which hydroxyl groups may be etherified or esterified).
7. Amide group-containing monomers: (meth) acrylamide, N-alkyl (meth)acrylamides (such as N-methylacrylamide, N-hexylacrylamide), N,N-dialkyl (meth)acryl amides (such as N,N-dimethylacrylamide, N,N'-di-n-propylacrylamide), N-hydroxyalkyl (meth)acrylamides [such as N-methylol(meth)acrylamide, N-hydroxyethyl (meth)acrylamide], N,N-dihydroxyalkyl (meth)acrylamides [such as N,N-dihydroxyethyl (meth)acrylamide], vinyl lactams (such as N-vinylpyrrolidone);
8. Amino group-containing monomers: amino group-containing esters (e.g. dialkylaminoalkyl esters, dihydroxyalkylaminoalkyl esters, morpholinoalkyl esters, etc.) of monoethylenically unsaturated mono- or di-carboxylic acid [such as dimethlaminoethyl (meth)acrylate, diethylaminoethyl (meth)acrylate, morpholinoethyl (meth)acrylate, dimethyl aminoethyl fumarate], heterocyclic vinyl compounds [such as vinyl pyridines (e.g. 2-vinyl pyridine, 4-vinyl pyridine, N-vinyl pyridine), N-vinyl imidazol]; and
9. Quaternary ammonium salt group-containing monomers: N,N,N-trialkyl-N-(meth)acryloyloxyalkylammonium salts [such as N,N,N-trimethyl-N-(meth)acryloyloxyethylammonium chloride, N,N,N-triethyl-N-(meth)acryloyloxyethylammonium chloride, 2-hydroxy-3-(meth)acryloyloxypropyl trimethyl ammonium chloride], and monomers as mentioned in British patent specification No. 1,034,296.

Suitable monomers which become water-soluble by hydrolysis, for use in this invention instead of or in conjunction with the water-soluble monomers, include monethylenically unsaturated compounds having at least one hydrolyzable group, such as ester and nitrile groups. Such monomers having an ester group include for example, lower alkyl (C1-C3) esters of monoethylenically unsaturated carboxylic acids, such as methyl (meth)acrylate, ethyl (meth)acrylate and 2-ethylhexyl (meth)acrylate; and esters of monoethylenically unsaturated alcohols [vinyl esters, (meth)-allyl ester, etc.], such as vinyl acetate and (meth) allyl acetate. Suitable nitrile group-containing monomers include (meth) acrylonitrile.

Preferred monomers include carboxylic acid monomers, or anhydrides or salts thereof. Especially preferred monomers include acrylic acid, methacrylic acid, and maleic acid, and anhydrides and salts thereof. Acrylic acid and combinations of acrylic acid and acrylate salt (e.g. sodium acrylate) are particularly preferred.

While at least 25 mole percent (preferably at least about 35%, more preferably at least about 50%) of the

hydrogel-forming polymer compositions herein should be prepared from acid group-containing monomers, some non-acid monomers may also be used to prepare the hydrogel-forming polymer compositions herein (prior to neutralization). It is particularly useful to incorporate non-acid monomers into polymers to be formed into fibers, in order to increase the flexibility of the final polymeric material. Such non-acid monomers can include, for example, the water-soluble or water-dispersible esters of the foregoing acid-containing monomers as well as monomers which contain no carboxyl or sulfonic acid groups at all. Optional non-acid monomers can thus include, for example, carboxylic acid or sulfonic acid ester-containing monomers e.g. $C_1$-$C_4$ esters, hydroxyl group-containing monomers, amide group-containing monomers, amino group-containing monomers, nitrile group containing monomers, quaternary ammonium salt group-containing monomers and unsaturated polymerzable hydrocarbons, such as alph-olefins, vinyl monomers, and vinylidene monomers. Examples include ethylene, propylene, isobutylene, 1-butylene, vinyl acetate, methyl vinyl ether, isobutyl vinyl ether, and styrene compounds of the formula:

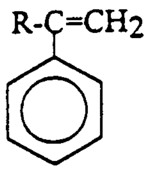

wherein R represents H or a $C_1$-$C_6$ alkyl, and wherein the benzene ring may be substituted with low molecular weight alkyls (e.g. $C_1$-$C_4$) or hydroxy groups. These non-acid monomers are well known materials and are described in greater detail, for example, in Masuda et al., U.S. Patent 4,076,663, issued February 28, 1978; and in Westerman, U.S. Patent 4,062,817, issued December 13, 1977; and U.S. Patent 5,151,465 Le-Khac, issued September 29, 1992. If present at all, such non-acid monomers should generally be used only to such an extent that, prior to neutralization, no more than 75% mole percent of the polymer compositions herein are prepared from such non-acid monomers, preferably no more than about 65%, more preferably no more than about 50%.

Especially preferred are copolymers of maleic acid, maleic anhydride, or malic acid salt with isobutylene or other $C_3$-$C_6$, preferably $C_4$, vinyl monomers most preferably isobutylene at a mole percent of from 35% to 65% maleic monomer units to 65% to 35% isobutylene or other $C_3$-$C_6$ vinyl monomers.

In the hydrogel-forming polymeric gelling agent the polymeric component formed from unsaturated, acid-containing monomers may be grafted on to other types of polymer moieties such as starch or cellulose.

Suitable starches include, for example, natural starches such as sweet potato starch, potato starch, wheat starch, corn starch, rice starch, tapioca starch, and the like, and processed or modified starches such as alpha-starch, dextrine, oxidized starch, dialdehyde starch, alkyl-etherified starch, allyl-etherified starch, oxyalkylated starch, aminoethyl-etherified starch, cyanoethyl-etherified starch and the like.

Suitable celluloses include, for example, celluloses obtained from wood, leaves, stems, bast, seed fluffs, and the like; and modified celluloses such as alkyl-etherified cellulose, organic-acid-esterified cellulose, oxidized cellulose, hydrocellulose, and the like. Starch grafted materials of this type are especially preferred for use herein.

Whatever the nature of the monomer components of the hydrogel-forming polymeric gelling agents used in the present compositions, the polymers thereof will be crosslinked. Suitable cross-linking agents are well know in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer material; and (4) polyvalent metal compounds which can form ionic cross-linkages.

Cross-linking agents having at least two polymerizable double bonds include (i) di- or polyvinyl compounds such as divinylbenzene and divinyltoluene; (ii) di- or poly-esters of unsaturated mono- or polycarboxylic acids with polyols including, for example, di- or triacrylic acid esters of polyols such as ethylene glycol, trimethylol propane, glycerine, or polyoxyethylene glycols; (iii) bisacrylamides such as N,N-methylenebisacrylamide; (iv) carbamyl esters that can be obtained by reacting polyisocyanates with hydroxyl group-containing monomers; (v) di- or poly-allyl ethers of polyols; (vi) di- or poly-allyl esters of polycarboxylic acids such as diallyl phthalate, diallyl adipate, and the like; (vii) esters of unsaturated mono- or poly-carboxylic acids with monoallyl esters of polyols such as acrylic acid ester of polyethylene glycol monoallyl ether; and (viii) di- or tri-allyl amine.

Cross-linking agents having at least one polymerizable double bond and at least one functional group reactive with the acid-containing monomer material include N-methylol acrylamide, glycidyl acrylate, and the like. Suitable cross-linking agents having at least two functional groups reactive with the acid-containing monomer material include glyoxal; polyols such as ethylene glycol and glycerol; polyamines such as alkylene diamines (e.g., ethylene diamine), polyalkylene polyamines, polyepoxides, di- or polyglycidyl ethers and the like. Suitable polyvalent metal cross-linking

agents which can form ionic cross-linkages include oxides, hydroxides and weak acid salts (e.g., carbonate, acetate and the like) of alkaline earth metals (e.g., calcium, magnesium) and zinc, including, for example, calcium oxide and zinc diacetate.

Cross-linking agents of many of the foregoing types are described in greater detail in Masuda et al., U.S. Patent 4,076,663, issued February 28, 1978, and Allen et al., U.S. Patent 4,861,539, issued August 29, 1989. Preferred cross-linking agents include the di- or polyesters of unsaturated mono- or polycarboxylic acids mono-allyl esters of polyols, the bisacrylamides, and the di- or tri-allyl amines. Specific examples of especially preferred cross-linking agents include N,N'-methylenebisacrylamide and trimethylol propane triacrylate.

The cross-linking agent will generally constitute from about 0.001 mole percent to 5 mole percent of the resulting hydrogel-forming polymeric material. More generally, the cross-linking agent will constitute from about 0.01 mole percent to 3 mole percent of the hydrogel-forming polymeric gelling agent used herein.

The hydrogel forming polymeric gelling agent herein is at least partially crosslinked, however the degree of crosslinking must be high enough such that the resulting polymer does not exhibit a glass transition temperature (Tg) below about 140°C, and accordingly, the term "hydrogel forming polymeric gelling agent," as used herein, shall mean polymers meeting this parameter. Preferably the hydrogel forming polymeric gelling agent does not have a Tg below about 180°C, and more preferably does not have a Tg prior to decomposition of the polymer, at temperatures of about 300°C or higher. The Tg can be determined by differential scanning calorimetry (DSC) conducted at a cooling rate of 20.0 C°/minute with 5 mg or smaller samples. The Tg is calculated as the midpoint between the onset and endset of heat flow change corresponding to the glass transition on the DSC heat capacity cooling curve. The use of DSC to determine Tg is well known in the art, and is described by B. Cassel and M. P. DiVito in "Use of DSC To Obtain Accurate Thermodynamic and Kinetic Data", American Laboratory, January 1994, pp 14-19, and by B. Wunderlich in Thermal Analysis, Academic Press, Inc., 1990.

The hydrogel forming polymeric gelling hereof are preferably employed in their partially neutralized form. For purposes of this invention, such materials are considered partially neutralized when at least 25 mole percent, and preferably at least 50 mole percent of monomers used to form the polymer are acid group-containing monomers which have been neutralized with a base. Suitable neutralizing bases cations include hydroxides of alkali and alkaline earth metal (e.g. KOH, NaOH), ammonium, substituted ammonium, and amines such as amino alcohols (e.g., 2-amino-2-methyl-1,3-propanediol, diethanolamine, and 2-amino-2-methyl-1-propanol. This percentage of the total monomers utilized which are neutralized acid group-containing monomers is referred to herein as the "degree of neutralization." The degree of neutralization will preferably not exceed 98%.

Hydrogel forming polymer gelling agent materials can be prepared by reacting monomers and cross-linking agents, as described above, in conventional manners. Prior art hydrogel forming polymeric gelling agent synthesis procedures are well known in the art and are also disclosed, for example, in the previously referenced U.S. Pat. Nos. 4,654,039, 4,286,082, and 4,340,706.

Suitable hydrogel forming polymeric gelling agents in the form of particles are commercially available from Hoechst Celanese Corporation, Portsmouth, VA, USA (Sanwet™ Superabsorbent Polymers) Nippon Shokubai, Japan (Aqualic™, e.g., L-75, L-76) and Dow Chemical Company, Midland, MI, USA (Dry Tech™).

Suitable hydrogel forming polymeric gelling agents in the form of fibers are commercially available from Camelot Technologies Inc., Leominster, MA, USA (Fibersorb™, e.g., SA 7200H, SA 7200M, SA 7000L, SA 7000, and SA 7300). Water

The compositions will comprise from 50% to 99.8%, by weight, water, preferably from 65% to 99.3%, more preferably from 80% to 99.2%. Water is an essential component in the present invention for forming the hydrogel thickening and suspending component.

Water Insoluble Silicone Emollient

The compositions hereof will contain from 0.1% to 15%, by weight, of water-insoluble silicone fluid as an emollient, preferably from 0.5% to 5%, more preferably from 0.5% to 2%. The silicone emollient hereof is dispersed throughout the composition as a discontinuous phase in the form of droplets, which are suspended by the hydrogel.

The number average particle size of the droplets containing the silicone emollient should be from 5 microns to 4,000 microns, in order for the compositions to be translucent. In order to further enhance translucent appearance, it is preferred that the average particle size be at least about 10 micron, preferably at least about 25 microns, more preferably at least about 40 microns. As droplet size become larger, they tend to refract less light and consequently the composition appears to have greater clarity. On the otherhand, as droplets size becomes larger, the droplets themselves remain clear, yet become visually distinguishable in the hydrogel matrix. Therefore, it is also preferable for the average droplet size be about 1000 microns or less, more preferably about 500 microns or less, most preferably about 250 microns or less. Particle size can be measured by a light scattering particle size analyzer.

The silicone fluids suitable for use herein as the silicone emollient include volatile and nonvolatile silicones.

The silicone fluids include polyalkylsiloxanes and polyalkyaryl siloxanes, including linear, branched chained, and cyclic silicones.

The silicone emollient component for use herein will generally have viscosity of from 0.5 to 2,000,000 centistokes at 25°C, more preferably from 0.5 to 1,000,000, even more preferably from 0.5 to 100,000. The viscosity can be measured by the ASTM D 445 standard method.

The polysiloxane fluid suitable for purposes hereof includes those represented by general formula:

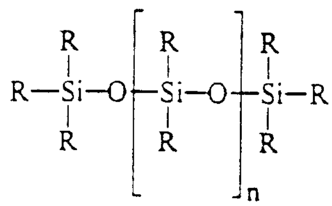

wherein each R independently is substituted or unsubstituted aliphatic (e.g. alkyl or alkenyl), aryl, aryloxy, alkaryl, alkamino (e.g. alkyl or alkenyl amino groups), hydroxy, or hydrogen, or combinations thereof; and n is zero or an integer, preferably from 1 to 10,000, more preferably from 1 to 1,000. The R substituents can also include combinations of hydroxy groups and amine groups, as well as other functional groups, such as halogens and halogen-substituted functionalities, e.g. halogen-substituted aliphatic and aryl groups. The structures can also include alkoxy functionalities, as long as the amount and type is not sufficient to impart significant surfactancy properties to the material.

Cyclic polysiloxanes include those represented by the general formula:

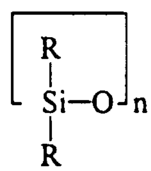

wherein R is as defined above, n is from 3 to 7, preferably from 3 to 5.

The substituents on the siloxane chain (R) may have any structure as long as the resulting polysiloxanes remain fluid at room temperature, are hydrophobic, are neither irritating, toxic nor otherwise harmful when applied to the skin, are compatible with the other components of the composition, are chemically stable under normal use and storage conditions, are capable of being deposited on the skin.

Preferred alkyl and alkenyl substitutes are $C_1$-$C_5$ alkyls and alkenyls, more preferably from $C_1$-$C_4$, most preferably from $C_1$-$C_2$. The aliphatic portions of other alkyl-, alkenyl-, or alkynyl- containing groups (such as alkaryl, and alkamino) can be straight or branched chains and preferably have from one to five carbon atoms, more preferably from one to four carbon atoms, even more preferably from one to three carbon atoms, most preferably from one to two carbon atoms. As discussed above, the R substituents hereof can also, contain amino functionalities, e.g., alkamino groups, which can be primary, secondary or tertiary amines or quaternary ammonium. These include mono-, di-, and tri-alkylamino and alkoxyamino groups wherein the aliphatic portion chain length is preferably as described above. The R substituents can also be substituted with other groups, such as halogens (e.g. chloride, fluoride, and bromide), halogenated aliphatic or aryl groups, and hydroxy (e.g. hydroxy substituted aliphatic groups). Suitable halogenated R groups could include, for example, tri-halogenated (preferably fluoro) alkyl groups such as $-R^1-C(F)_3$, wherein $R^1$ is $C_1$-$C_3$ alkyl. Examples of such polysiloxanes include polymethyl -3,3,3 trifluoropropylsiloxane.

Aryl-containing substituents contain alicyclic and heterocyclic five and six membered aryl rings, and substituents containing fused five or six membered rings. The aryl rings themselves can be substituted or unsubstituted. Substituents include aliphatic substituents, and can also include alkoxy substituents, acyl substituents, ketones, halogens (e.g., Cl and Br), amines, etc. Exemplary aryl-containing groups include substituted and unsubstituted arenes, such as phenyl, and phenyl derivatives such as phenyls with $C_1$-$C_5$ alkyl or alkenyl substituents, e.g., allylphenyl, methyl phenyl and ethyl phenyl, vinyl phenyls such as styrenyl, and phenyl alkynes (e.g. phenyl $C_2$-$C_4$ alkynes). Heterocyclic aryl groups include substituents derived from furan, imidazole, pyrrole, pyridine, etc. Fused aryl ring substituents include, for example, napthalene, coumarin, and purine.

For purposes hereof, volatile shall mean materials with a vapor pressure at 25°C of 0.2mm Hg or higher. Non-volatile shall mean materials with a vapor pressure of less than 0.1 mm Hg at 25°C. In general, the preferred cyclomethicones will be volatile and dimethicones having a viscosity at 25°C of about 10 centipoise or higher will be non-volatile.

Substantially Free of Surfactants

The compositions of the present invention are substantially free of surfactants. By "substantially free", in regard to the absence of surfactants, what is meant is that the compositions will contain no more than about 0.1%, by weight, of surfactants, preferably no more than about 0.05%, more preferably no more than about 0.01%, most preferably 0%.

Insofar as the compositions hereof are substantially free of surfactants, it should be understood that silicone surfactants, such as the dimethicone copolyol surfactants, e.g. dimethiconol, are not intended to be included within the silicone emollients hereof.

Composition Viscosity

The compositions of the present invention will have a sufficient amount of hydrogel forming polymeric gelling agent such that the composition has an average viscosity of at least about 4,000 centipoise, at 25°C, as measured using a Brookfield RVT Viscometer equipped with a helipath stand using a 1.435 inch (3.65 cm) T-spindle at 5.0 revolutions/minute, with a plunge rate of 2.3 cm/minute and the measurement taken after a 2.0 minute plunge (i.e., 2.0 minutes after initial contact of the revolving spindle with the top surface of the product), or equivalent. The average viscosity is preferably from 10,000 to 35,000 centipoise.

Optional Ingredients

The present compositions can comprise a wide variety of optional ingredients. Such optional ingredients can be used, for example, for aesthetic purposes, cosmetic purposes, medicinal purposes, skin-protection purposes, or processing ease. Exemplary optional materials are described below. When used, optional ingredients should be present at levels sufficient to provide the intended benefit. In general they will be added at a level of about 0.05%, or higher depending upon the efficacy of the particular ingredient.

Perfume

The compositions of the present invention will optionally, and preferably, comprise from 0.01% to 5%, by weight of a perfume, preferably from 0.05% to 3%, more preferably from 0.1% to 1%. Water soluble and water insoluble perfumes can be used, although water insoluble perfumes are generally preferred since they provide much greater variety and flexibility to the perfumer. In a particularly preferred embodiment hereof, the water insoluble perfume is combined in the water insoluble silicone emollient droplet phase of the present invention, whereby it is suspended in the composition along with the emollient. When combining the perfume and silicone emollient into the same dispersed phase, it is preferred for the silicone fluid used in the emollient phase to have a viscosity of about 50 centistokes or less, at 25°C, to enhance miscibility of the perfume and silicone.

By water insoluble perfume, what is meant is that the perfume forms a distinct phase in deionized water at 25°C in the absence of a surfactant or other solubilizer. Water insoluble perfumes will, in general, either separate to form a distinct phase or form a turbid solution in deionized water, at 25°C, at a concentration of 1.0%, by weight, perfume.

Water soluble perfumes, on the otherhand, are those perfumes which are not water insoluble as herein defined. By perfume, what is meant is a volatile, odoriferous, component of one or more active perfume compounds which exudes a pleasant or otherwise desired aroma at ambient conditions, or which mask odors. The perfume comprises odoriferous, water-insoluble perfume active compounds and may also include additional ingredients, such as diluents, solvents for solid perfume ingredients, and fixatives, etc.

The perfumes hereof are, in general, liquids at ambient temperature and are characterized by a flash point of from 10°C to

120°C, more typically from 25°C to 95°C (as determined according to ASTM D-56 (c.c.) - Standard Test Method for Flash Point by Tag Closed Tester).

The perfume active compounds are typically incorporated into the perfume components in liquid form, but can also be solids (such as the various camphoraceous perfumes known in the art) which are solubilized in other ingredients of the perfume component.

As discussed, in addition to the perfume active compounds, the perfume hereof can also include additional ingredients such as diluents, solvents for solid perfume ingredients, and fixatives, etc. Diluents may or may not have their own aroma and, to the extent that they do, they are categorized as perfume active compounds. Exemplary diluents and solvents include alcohols (e.g. ethyl alcohol, benzyl alcohol, dipropylene glycol, etc.) and liquid hydrocarbon and hydrocarbon esters (e.g., benzyl benzoate and other hydrocarbons and esters described above). Fixatives are ingredients which prolong the lasting quality of the perfume upon use and can do so by modifying the overall volatility of the perfume component. Some fixatives can function as perfume active compounds, whereas others do not. To the extent that a particular ingredient performs both functions, it shall be considered a perfume active compound. Exemplary fixatives include musk perfume ingredients described below.

A wide variety of perfume active compounds are described in S. Arctander, Perfume Flavors and Chemicals, Vols, I and II., Aurthor, Montclair, NJ, the Merck Index, 8th Edition, Merck & Co., Inc., Rahway, NJ, and Secondini, Handbook of Perfumes and Flavors, Chemical Publishing Co., Inc., New York, NY, 1990 (ISBN) 0-8206-0334-1).

The typical perfume will comprise a plurality of individual perfume active compounds, although it can consist essentially of a single perfume ingredient. It is well within the scope of the perfumer of ordinary skill in the art changing ingredients in the perfume component and/or modifying the relative levels of perfume ingredients.

Various types of chemical compounds are commonly known for perfumery uses including: phenolic compounds; essential oils; aldehydes; ketones; polycyclic compounds; esters; and alcohols. Many perfume ingredients contain a combination of functional groups and can be categorized under two or more of the above classes.

From the standpoint of the perfumer, it is convenient to consider the perfume ingredients in terms of the type of aroma it imparts rather than the particular chemical class or classes it may fall within. The perfume components herein can be formulated to provide a variety of odor categories: a non-exclusive list includes woody, sweet, citrus, floral, fruity, animal, spice, green, musk, balsamic, chemical, and mint. A variety of exemplary perfume ingredients are described below for several of the commonly used odor categories, long with their representative (but not necessarily exclusive) chemical categories.

Woody perfume ingredients include cedarwood oil (essential oil), guaicwood oil (essential oil), gamma ionone (ketone), sandalwood oil (essential oil), and methyl cedrylone (ketone).

Sweet perfume ingredients include coumarin (ketone), vanillin (4 hydroxy-3methoxy benzaldehyde) (aldehyde), ethyl maltol (Alcohol), phenyl acetaldehyde (aldehyde), heliotropin (aldehyde), acetophenone (ketone), and dihydrocoumarin (ketone).

Citrus perfume ingredients include orange oil (essential oil), lemon oil (essential oil), citral (aldehyde), beta methyl naphthyl ketone (ketone), terpinyl acetate (ester), nonyl aldehyde (aldehyde), terpineol (alcohol), and dihydromyrcenol (alcohol).

Floral perfume ingredients include a variety of floral subcategories, such as rose, lavender, jasmin, and muguet. Rose perfume ingredients include geranyl acetate (ester), geraniol (alcohol), citronelyl acetate (ester), phenyl ethyl alcohol (alcohol), alpha damascone (ketone), beta damascone (ketone), geranium oil (essential oil), and natural rose oil (essential oil). Lavender perfume ingredients include dihydro terpinyl acetate (ester), ethyl hexyl ketone (ketone), lavandin (essential oil), lavender (essential oil), tetra hydro linalool (alcohol), linalool (alcohol), and linalyl acetate (ester). Jasmin perfume ingredients include benzyl acetate (ester), butyl cinnamic aldehyde (aldehyde), methyl benzoate (ester), natural jasmin oil (essential oil), methyl dihydro jasmonate (ester). Muguet perfume ingredients include cycalmen aldehyde (aldehyde), benzyl salicylate (ester), hydroxycitronellol (alcohol), citronellyl oxyacetaldehyde (aldehyde), and hydroxy aldehyde (aldehyde).

Fruity perfume ingredients include ethyl-2-methyl butyrate (ester), ally cyclohexane propionate (ester), amyl acetate (ester), ethyl acetate (ester), gamma decalactone (ketone), octalactone (ketone), undecalactone (aldehyde), ethyl aceto acetate (ester), benzaldehyde (aldehyde).

Animal perfume ingredients include methyl phenyl acetate (ester), indol (2,3, benzpyrrole) (phenolic), creosol (phenolic), iso butyl quinolin (phenolic), and androstenol (phenolic).

Spice perfume ingredients include anisic aldehyde (aldehyde), anise (essential oil), clove oil (essential oil), eugenol (phenolic), iso eugenol (phenolic), thymol (phenolic), anethol (phenolic), cinnamic alcohol (alcohol), and cinnamic aldehyde (aldehyde).

Green perfume ingredients include beta gamma hexenol (alcohol), brom styrol (alcohol), dimethyl benzyl carbinol (alcohol), methyl heptine carbonate (ester), cis-3-hexenyl acetate (ester), and galbanum oil (essential oil).

Musk perfume ingredients often also function as fixatives. Examples of musk include glaxolide (phenol), cyclopentadecanolide (phenol), musk ketone (ketone), ambrettolide (phenol), tonalid (phenol), and ethylene brassylate (ester).

Balsamic perfume ingredients include fir balsam (essential oil), peru balsam (essential oil), and benzoin resinoid (essential oil).

Chemical perfume ingredients include benzyl alcohol (alcohol), dipropylene glycol (alcohol), ethanol (alcohol), and benzyl benzoate (ester).

Mint perfume ingredients include laevo carvone (ketone), menthol (alcohol), methyl salicylate (ester), peppermint oil (essential oil), spearmint oil (essential oil), eucalyptus (essential oil), anisyl acetate (ester), methyl chavicol (alcohol).

Additional Conditioning Agents

Additional conditioning agent can also be included in the present compositions. Optional conditioning agents suitable for use include both humectants and non-silicone emollients. In general, such additional conditioning agents are used at levels of up to about 20%, by weight of the composition, preferably up to about 10%. When added to provide functional benefits, they will generally be added at a level of at least about 0.05%, preferably at least about 0.1%.

Exemplary conditioning agents include $C_3$-$C_6$ diols and triols, e.g., propylene glycol, hexylene glycol, 1,3-dihydroxypropane, butylene glycol, 1,4-dihydroxyhexane, 1,2,6-hexane triol, glycerin, and the like.

Other conditioning agents include: other polyhydroxy alcohols, such as sorbitol; sugars and starches, and derivatives thereof, such as alkoxylated sugars and starches, e.g., alkoxylated glucose; aloe vera; panthenol; hyaluronic acid; lactamide monoethanolamine; octamide monoethanolamine; polyethylene glycol polymers (PEG), polypropylene glycol polymers (PPG), and copolymers of PEG and PPG (e.g., PEG/PPG 17/6 copolymer).

Water-insoluble emollient conditioning agents, including hydrocarbons, fatty esters and alcohols, and the like can also be used in addition to the silicone emollient. The average particle size of dispersed phases containing them should preferably be within the ranges provided above.

Sensates and Coolants

A wide variety of coolants and sensate materials can be used. Sensates include menthol, acyclic carboxamides such as those described in U.S. Patent 4,230,688, Rowsell et al., issued October 28, 1980, e.g., N,2,3-trimethyl-2-isopropylbutanamide, and N-substituted-p-menthane-3-carboxamides such as those described in U.S. Patent 4,136,163, Watson et al., issued January 23, 1979, e.g., N-ethyl-p-menthane-3-carboxamide. Sensates are generally used at levels of from 0.01%, to 2%, by weight, preferably from 0.05% to 1.0%, more preferably from 0.95% to 0.5%. Coolants include volatile liquids that evaporate on the skin, thereby resulting with a cooling sensation. Preferred coolants include ethanol, isopropyl alcohol, and witch hazel. The level of such coolants can vary widely. In general, at least about 0.5%, by weight, will be added if used for cooling purposes, preferably at least about 1%. Maximum levels are governed by practical concerns and the degree of cooling sensation desired, as well as the desire to minimize possible stinging, and will generally be no more than about 40%, more generally no more than about 30%.

Skin Renewal Agents

Skin renewal agents can also be used in the present compositions, typically at levels of from 0.05% to 5%, by weight, preferably from 0.1% to 2%. These include alpha-hydroxy acids, such as glycolic acid, and glutaric acid, salicylic acid, lactic acid, and other fruit acids.

Additional Components

The compositions of the present invention can comprise a wide range of additional components. Examples of these classes of additional components include: anti-acne agents, anti-foaming agents, anti-microbial agents, antioxidants, binders, biological additives, buffering agents, chelating agents, colorants, cosmetic astringents, cosmetic biocides, denaturants, drug astringents, external analgesics, film formers, fragrance components, pH adjusters, preservatives, reducing agents, skin bleaching agents, sunscreen agents (e.g., ultraviolet light absorbers); solvents such as monohydric $C_1$-$C_4$ alcohols (typically at levels from 0% to about 40%, preferably from 0% to about 20%, more preferably from 0% to about 5%), especially ethanol; etc.

Other nonlimiting examples of these additional components include the following: vitamins and derivatives thereof (e.g., tocopherol, tocopherol acetate, retinoic acid, retinol, retinoids, and the like); polymers for aiding the film-forming properties and substantively of the composition (such as the copolymer of eicosene and vinyl pyrrolidone, an example of which is available from GAF Chemical Corporation as Ganex® V-220); preservatives for maintaining the anti-microbial integrity of the compositions; other anti-acne medicaments (e.g., resorcinol, sulfur, salicylic acid, erythromycin, and the like); and skin bleaching (or lightening agents including but not limited to hydroquinone, kojic acid, antioxidants, chelators and sequestrants.

PROCESS FOR MAKING

The compositions hereof can be made by mixing the ingredients together according to conventional techniques known in the art. In general, the compositions can be made by combining water and the hydrogel forming polymeric gelling agent, mixing, and adding the other water soluble ingredients. The water insoluble phase can then be added with mixing. Medium or high shear mixing may be used to adjust emollient phase particle size to the desired level.

METHOD OF USE

The compositions hereof can be used by topically applying an effective amount for conditioning the skin, such as via spreading with the hands or an article, or by spraying. In general, from 0.05 to 10 g per square centimeter of skin is applied, preferably from 0.05 g to 3 g per square centimeter.

EXPERIMENTAL

A) Absorptive Capacity "Tea Bag" Test

Absorptive Capacity can be determined by a gravimetric analytical technique using deionized water as the fluid for which Absorptive Capacity of the polymeric gelling agent is to be calculated. A sample of polymeric gelling agent is placed within a tea bag, immersed in an excess of deionized water for a specified period of time, and then centrifuged for a specific period of time. The ratio of polymeric gelling agent final weight after centrifuging minus initial weight (net fluid gain) to initial weight determines the Absorptive Capacity.

The following procedure is conducted under standard laboratory conditions at 23°C (73°F) and 50% relative humidity. Using a 6 cm X 23 cm cutting die, the tea bag material is cut, folded in half lengthwise and sealed along two sides with a T-bar sealer to produce a 6 cm X 6 cm tea bag square. The tea bag material utilized is a grade 1234 heat sealable material, obtainable from C. H. Dexter, Division of the Dexter Corp., Windsor Locks) Connecticut, U.S.A, or equivalent. Lower porosity tea bag material should be used if required to retain fine particles or fibers of polymeric gelling agent. 0.200 grams plus or minus 0.005 grams of the polymeric gelling agent is weighed onto a weighing paper and transferred into the tea bag and the top (open end) of the tea bag is sealed. An empty tea bag is sealed at the top and is used as a blank. Approximately 300 milliliters of deionized water are poured into a 1,000 milliter beaker. The blank tea bag is submerged in the deionized water. The tea bag containing the polymeric gelling agent (the sample tea bag) is held horizontally to distribute the material evenly throughout the tea bag. The tea bag is laid on the surface of the deionized water. The tea bag is allowed to wet, for a period of no more than one minute, and then is fully submerged and soaked for 60 minutes. Approximately 2 minutes after the first sample is submerged, a second set of tea bags, prepared identically to the first set of blank and sample tea bags, is submerged and soaked for 60 minutes in the same manner as the first set. After the prescribed soak time has elapsed, for each set of tea bag samples, the tea bags are promptly removed (using tongs) from the deionized water. The samples are then centrifuged as described below. The centrifuge used is a Delux Dynac II Centrifuge, Fisher Model No. 05-100-26, obtainable from the Fisher Scientific Co. of Pittsburgh, PA, or equivalent. The centrifuge should be equipped with a direct read tachometer and an electric brake. The centrifuge is further equipped with a cylindrical insert basket having an approximately 2.5 inch (6.35 cm) high outer wall with an 8.435 inch (21.425 cm) outer diameter, a 7.935 inch (20.155 cm) inside diameter, and 9 rows each of approximately 106 3/32 inch (0-238 cm) diameter circular holes equally spaced around the circumference of the outer wall, and having a basket floor with six 1/4 inch (0.635 cm) diameter circular drainage holes equally spaced around the circumference of the basket floor at a distance of 1/2 inch (1.27 cm) from the interior surface of the outer wall to the center of the drainage holes, or an equivalent. The basket is mounted in the centrifuge so as to rotate, as well as brake, in unison with the centrifuge. The-sample tea bags are positioned in the centrifuge basket with a folded end of the tea bag in the direction of the centrifuge spin to absorb the initial force. The blank tea bags are placed to either side of the corresponding sample tea bags. The sample tea bag of the second set must be placed opposite the sample tea bag of the first set; and the blank tea bag of the second set opposite the blank tea bag of the first set, to balance the centrifuge. The centrifuge is started and allowed to ramp up quickιy to a stable speed of 1,500 rpm, a timer is set for 3 minutes. After 3 minutes, the centrifuge is turned off and the brake is applied. The first sample tea bag and the first blank tea bag are removed and weighed separately. The procedure is repeated for the second sample tea bag and the second blank tea bag.

The Absorptive Capacity (AC) for each of the samples is calculated as follows: AC = (sample tea bag weight after centrifuge minus blank tea bag weight after centrifuge minus polymeric gelling agent weight) / (dry polymeric gelling agent).

The Absorptive Capacity value for use herein is the average absorptive capacity of the two samples.

B) Extractable Polymer Content Determination

Depending upon the type of hydrogel-forming crosslinked polymeric gelling agent involved, two different methods are used herein to calculate extractable polymer content. For carboxylic acid-based polymeric gelling agent a potentiometric procedure is used to determine extractables. For sulfonic acid-based polymeric gelling agent, a gravimetric procedure is employed. It should be noted that both of these procedures may provide results that include in the total amount of extractable material those extractable components in the polymeric gelling agent which are not polymeric. Therefore, if a given polymer sample is known or believed to contain significant amounts of non-polymeric extractable material, such non-polymeric extractable material should be removed from the analyte in conventional fashion before running the extractable polymer content determination hereinafter described.

(1) Carboxylic Acid-Based Polymeric Gelling Agent

Extractable polymer content of carboxylic acid-based polymeric gelling agent is determined by admixing the polymeric gelling agent with deionized water for a period of time sufficient to substantially approach equilibrium with respect to extraction of polymer material from the hydrogel which is formed. The hydrogel/deionized water mixture is allowed to settle and a portion thereof is filtered. An aliquot of this filtrate is then taken, and the free acid groups on the polymer material dissolved in this filtrate are titrated to pH 10 with base. All of the carboxylate groups are then titrated to pH 2.7 with acid. These titration data are then used to calculate the amount of extractable polymer in the polymeric gelling agent sample.

(a) Preparation of the Extractable Polymer-Containing Filtrate Samples

1. 0.40 to 0.41 g of polymeric gelling agent is accurately (to ± 0.1 mg) weighed into a 150 ml disposable beaker. If glass beakers are used they must be acid washed prior to use. (Glassware should be washed three times with dilute HCl [conc. HCl diluted 1:4 with dieionized water], then three times with deionized water. This procedure removes trace of detergents and other contaminants which would otherwise interfere with the titration.)
2. 75 ml of deionized water are added.
3. Samples are slowly stirred for a period of time sufficient to reach equilibrium. Equilibrium is generally reached within 16 hour periods. If extractable polymer content is to be measured as a function of time, then 1, 6, and 16 hour periods are generally sufficient to define the extractables versus time curve.
4. Samples are allowed to settle for 15 minutes.
5. Using a 3 ml disposable syringe and 0.22 micron filters, enough solution is filtered so that a 20 ml aliquot can be taken.

(b) Titration Conditions

1. If the titrations are to be performed manually, great care must be taken to assure that equilibrium is reached after each addition of titrant.
2. A 20 ml aliquot of the filtrate is transferred to a 50 ml disposable beaker. If glass beakers are being used, they must be acid washed prior to use as noted herein before.
3. The aliquot is titrated to pH 10 with 0. 1N NaOH.
4. The aliquot is then back titrated to pH 2.7 with 0. 1N HCl.
5. Step 3 and 4 are performed on 20 ml of deionized water to obtain titration blinks for both steps of the titration.

(c) Calculations

1. The amount of polymerized acid moieties (e.g., acrylic acid) (in millimoles) in the supernatant aliquot ($M_a$) is given by:

$$M_a = (V_a - V_{ab}) \times N_a \text{ millimoles (mm)}$$

where:

$V_a$= The volume (in ml) of acid required to titrate the aliquot to pH 10.;
$V_b$= The volume (in ml) of acid required to titrate 20 ml of deionized water to pH 10; and
$N_a$= The normality (in meq/ml) of the acid (nominally 0.10 meq/ml)

2. The total amount of polymerized acid moieties (e.g. acrylic acid) plus polymerized neutralized acid moieties (e.g.,

sodium acrylate) (in mm) in the supernatant aliquot ($M_t$) is given by:

$$M_t=(V_b-V_{bb})xN_b \text{ millimoles}$$

where:

$V_b$= The volume (in ml) of base required to titrate the aliquot from pH 10 down to pH 2.7;
$V_{bb}$= The volume (in ml) of base required to titrate 20 ml of deionized water from pH 10 down to pH 2.7; and
$N_b$ = The normality (in meq/mi) of the base (nominally 0.10 meq/ml).

3. The amount of polymerized neutralized acid moieties (e.g., sodium acrylate) (in mm) in the original supernatant aliquot ($M_b$) is given by:

$$M_b=M_t-M_a$$

4. The total amounts of polymerized acid moieties ($W_a$) and polymerized neutralized acid moieties($W_b$) (e.g., acrylic acid plus sodium acrylate) extracted (in mg) are given by:

$$W_a=M_axE_axD \text{ and } W_b=M_bxE_{bx}D$$

where:

$E_a$ = The equivalent weight of add moiety in polyacid moiety (e.g., acrylic acid in polyacrylic acid =72 meq/mg).
$E_b$= The equivalent weight of neutralized acid moiety in neutralized polyacid moiety (e.g., sodium acrylate in sodium polyacrylate = 94 meq/mg).
D = The dilution factor (75 ml/20 ml= 3.75).

5. The percent extractable polymer in the polymeric gelling agent sample (e) is given by:

$$e=((W_a+W_b) \text{ x } 100)/W$$

where:

W= The sample weight in mg.

2. Sulfonic Acid-Containing Polymeric Gelling Agent

Extractable polymer content of sulfonic acid-based polymeric gelling agent is determined by a gravimetric procedure wherein hydrogel samples are swollen overnight in deionized water, and the polymer content in the nitrate is gravimetrically determined. The particular procedure of the gravimetric extractables determination are set forth as follows:

Into a 500 ml Erlenmeyer flask is weighed accurately (to +/- 0.1 mg) about 0.25 grams of dry polymeric gelling agent ($W_p$). 250 ml of deionized water is added and the mixture is stirred slowly for 1 hour. After this hour has passed, stirring is stopped, and the swollen gel is allowed to settle for about 16 hours. The supernatant is filtered using a 3 ml disposable syringe and 0.22 micron filter to obtain at least 40 ml of filtrate. Exactly 40 ml of filtrate is placed into a clean 100 ml round-bottomed flask, and the solution is concentrated on a rotary evaporator (water aspirator vacuum, bath temperature 55°C). The remaining 2-3 ml of solution is transferred quantitatively to a tared weighing vial with the aid of additional deionized water. The solution in the weighing vial is reduced to dryness in an oven at 120°C. The vial is cooled, reweighed, and the weight of residue ($W_r$) is determined using the tare weight of the vial. The percent extractable polymer (e) is calculated from the weight of dry polymer ($W_p$) and weight of residue ($W_r$) by the following equation:

$$e = (W_r \text{ x } 250 \text{ x } 100)/(W_p \text{ x } 40)$$

EXAMPLES

Below are examples of the present invention which are provided for purposes of exemplifying specific embodiments of the invention, and are not intended to necessarily limit the invention thereto. The scope of the invention is defined in the claims which follow.

| Ingredient (wt. %) | 1 | 2 | 3 | 4 | 5 |
|---|---|---|---|---|---|
| Water | 97.20 | 82.75 | 89.93 | 87.23 | 88.63 |
| Hydrogel Forming Polymeric Gelling Agent[1] | 0.50 | 0.35 | 0.40 | 0.55 | 0.50 |
| Silicone[2] | 1.50[a] | 5.00[b] | 2.25[c] | 1.00[a] | 1.50[b] |
| Triethanolamine | 0.05 | 0.05 | 0.05 | 0.05 | 0.05 |
| Glycerin | - | 6.00 | 2.50 | 3.00 | 1.50 |
| Dexpanthenol | - | 2.00 | 2.50 | 3.00 | 3.00 |
| PEG/PPG-17/6 Copolymer[3] | - | 2.00 | 1.00 | 3.50 | 2.50 |
| Perfume | - | 1.00 | 0.50 | 0.75 | 1.50 |
| Sensate[4] | - | 0.08 | 0.10 | 0.15 | 0.05 |
| Disodium EDTA | - | 0.02 | 0.02 | 0.02 | 0.02 |
| DMDM Hydantoin (and) Iodopropynyl Butylcarbamate[5] | 0.20 | 0.20 | 0.20 | 0.20 | 0.20 |
| Color Sol'n (0.2% aqueous solution) | 0.50 | 0.50 | 0.50 | 0.50 | 0.50 |
| Benzophenone-2 | 0.05 | 0.05 | 0.05 | 0.05 | 0.05 |

1 Sanwet IM-1000 (Hoechst Celanese/Fine Chemicals)
2
[a] DC 200 Fluid, 5 cs, Dimethicone (Dow Corning)
[b] DC 345 Fluid, Cyclomethicone (Dow Corning)
[c] DC 200 Fluid, 50 cs, Dimethicone (Dow Corning)
3 UCON 75-H-450 (Union Carbide)
4 N,2,3-trimethyl-2-isopropyl-butanamide, or N-ethyl-p-menthane-3-carboxamide (Sterling Organics, Limited)
5 Glydant Plus[TN], (Lonza, Incorporated)

The above formulations are made as follows. Seventy to eighty percent of the total required water is added to a mixing vessel equipped with a rotor-stator homogenizer. The hydrogel forming polymeric gelling agent is added with mixing at 9500 revolutions per minute (rpm), at a rate of approximately 3 grams/minute. The mixing speed is then increased to 24,000 rpm for five minutes. A clear hydrogel is formed (Premix A). In a separate container equipped with a three blade stirrer, 10-15% of the total required water is added. Mixing is begun at 500 rpm and the water soluble components added (Triethanolamine, Glycerin, Dexpanthenol, PEG/PPG-17/6 Copolymer, Disodium EDTA, Glydant Plus[TN], Color Solution, Benzophenone-4). Mixing is continued until the solution is clear (Premix B). In another separate mixing vessel equipped with a three blade stirrer, the water insoluble materials are added (Perfume, Silicone, Sensate). Mixing is continued until all solids are dissolved (Premix C). Premix B is added to Premix A at a rate of about 1 gram/second with stirring. Mixing should continue until homogenous. Premix C is then added slowly (at about 10 grams/minute) with stirring. Particle size of the dispersed phases may be decreased by further mixing with a three blade mixer or a homogenizer.

**Claims**

**1.** A translucent, hydrogel, topical skin care composition comprising:

a) from 0.1% to 10% by weight, hydrogel forming polymeric gelling agent, calculated on a dry polymer weight basis, wherein said gelling agent is a crosslinked polymer having an absorptive capacity of at least about 40 grammes of water per gramme of gelling agent and not having a Tg below 140°C.
b) from 0.1% to 15%, preferably from 0.2% to 5%, by weight water-insoluble silicone emollient: said emollient being in the form of droplets having a number average particle size of from 5 microns to 4,000 microns, preferably from 10 microns to 1,000 microns, more preferably from 25 microns to 500 microns: and
c) from 50% to 99.8% preferably from 65% to 99.3%, by weight water; wherein said composition is clear and has a viscosity at 25°C of at least 4,000 centipoise and is substantially free of surfactants.

2. A skin care composition as in Claim 1, wherein said hydrogel forming polymeric gelling agent is a crosslinked polymer having an absorptive capacity of at least about 60 grammes of water per gramme of gelling agent and does not have a $T_g$ below 180°C, and preferably does not have a $T_g$ prior to decomposition of said polymeric gelling agent at a temperature of 300°C or higher.

3. A skin care composition as in Claim 2, wherein said hydrogel forming polymeric gelling agent is a crosslinked polymer having an absorptive capacity of at least about 80 grammes of water per gramme of gelling agent and does not have a $T_g$ below 180°C, and preferably does not have a $T_g$ prior to decomposition of said polymeric gelling agent at a temperature of 300°C or higher.

4. A skin care composition as in Claim 2, wherein said hydrogel forming polymeric gelling agent is a crosslinked polymer comprising monoethylenically unsaturated carboxylic acid monomers, or anhydrides or salts thereof.

5. A skin care compositions as in Claim 2, wherein said hydrogel forming polymeric gelling agent is selected from the group consisting of polyacrylic acid polymer, polymethacrylic acid polymer, polyacrylic acid/polymethacrylic acid copolymer, polymers derived from anhydrides and salts of acrylic acid and methacrylic acid and mixtures thereof.

6. A skin care composition as in Claim 2, wherein said composition comprises from 0.5% to 5%, preferably from 0.2% to 5%, by weight, of said emollient which is selected from the group consisting of volatile silicones, nonvolatile silicones, and mixtures thereof, and from 65% to 99.3%, preferably from about 65% to 99.2% by weight, water.

7. A skin care composition as in Claim 2, wherein said composition comprises:

   a) from 0.3% to 1%, by weight, of said hydrogel forming polymeric gelling agent;
   b) from 0.5 % to 2% by weight, of said silicone emollient, wherein said emollient is present in the form of droplets having a number average particle size of from 40 microns to 250 microns; and
   c) from 80% to 99.2%, by weight, water;

   wherein said composition has a viscosity at 25°C of from 7,000 centipoise to 35,000 centipoise.

8. A skin care composition as in Claim 2, further comprising water insoluble perfume incorporated into said droplets of component (b), wherein said silicone emollient has a viscosity at 25°C of 50 centistokes or less.

**Patentansprüche**

1. Transluzente, hydrogelförmige, topische Hautpflegezusammensetzung, umfassend:

   (a) 0,1 bis 10 Gew.-% eines hydrogelbildenden, polymeren Gelierungsmittels, berechnet auf Trockenpolymer-Gewichtsbasis, wobei das Gelierungsmittel ein vernetztes Polymer mit einer Absorptionskapazität von mindestens etwa 40 Gramm Wasser pro Gramm Gelierungsmittel ist und keinen Tg unterhalb 140°C aufweist;
   (b) 0,1 bis 15, vorzugsweise 0,2 bis 5 Gew.-%, wasserunlösliches Silicon-Erweichungsmittel, wobei das Erweichungsmittel in Form von Tropfen mit einer zahlenmittleren Teilchengröße von 5 µm bis 4.000 µm, vorzugsweise 10 µm bis 1.000 µm, weiter vorzugsweise 25 µm bis 500 µm, vorliegt; und
   (c) 50 bis 99,8, vorzugsweise 65 bis 99,3 Gew.-%, Wasser;

   wobei die Zusammensetzung klar ist und eine Viskosität bei 25°C von mindestens 4.000 centipoise aufweist und im wesentlichen frei an Tensiden ist.

2. Hautpflegezusammensetzung nach Anspruch 1, wobei das hydrogelbildende, polymere Gelierungsmittel ein vernetztes Polymer mit einer Absorptionskapazität von mindestens etwa 60 Gramm Wasser pro Gramm Gelierungsmittel ist und keinen Tg unterhalb 180°C aufweist, und vorzugsweise keinen Tg vor der Zersetzung des polymeren Gelierungsmittels bei einer Temperatur von 300°C oder höher aufweist.

3. Hautpflegezusammensetzung nach Anspruch 2, wobei das hydrogelbildende, polymere Gelierungsmittel ein vernetztes Polymer mit einer Absorptionskapazität von mindestens etwa 80 Gramm Wasser pro Gramm Gelierungsmittel ist und keinen Tg unterhalb 180°C aufweist, und vorzugsweise keinen Tg vor der Zersetzung des polymeren Gelierungsmittels bei einer Temperatur von 300°C oder höher aufweist.

4. Hautpflegezusammensetzung nach Anspruch 2, wobei das hydrogelbildende, polymere Gelierungsmittel ein vernetztes Polymer ist, umfassend monoethylenisch ungesättigte Carbonsäuremonomere oder Anhydride oder Salze hiervon.

5. Hautpflegezusammensetzung nach Anspruch 2, wobei das hydrogelbildende, polymere Gelierungsmittel aus der Polyacrylsäurepolymer, Polymethacrylsäurepolymer, Polyacrylsäure/Polymethacrylsäure-Copolymer, von Anhydriden und Salzen von Acrylsäure und Methacrylsäure abgeleitete Polymere und Mischungen hiervon umfassenden Gruppe gewählt ist.

6. Hautpflegezusammensetzung nach Anspruch 2, wobei die Zusammensetzung 0,5 bis 5, vorzugsweise 0,2 bis 5 Gew.-%, des Erweichungsmittels umfaßt, das aus der flüchtige Silicone, nichtflüchtige Silicone und Mischungen hiervon umfassenden Gruppe gewählt ist, sowie 65 bis 99,3, vorzugsweise etwa 65 bis 99,2 Gew.-% Wasser.

7. Hautpflegezusammensetzung nach Anspruch 2, wobei die Zusammensetzung umfaßt:

   (a) 0,3 bis 1 Gew.-% des hydrogelbildenden, polymeren Gelierungsmittels;
   (b) 0,5 bis 2 Gew.-% des Silicon-Erweichungsmittels, wobei das Erweichungsmittel in Form von Tropfen mit einer zahlenmittleren Teilchengröße von 40 µm bis 250 µm vorliegt; und
   (c) 80 bis 99,2 Gew.-% Wasser;

   wobei die Zusammensetzung eine Viskosität bei 25°C von 7.000 centipoise bis 35.000 centipoise aufweist.

8. Hautpflegezusammensetzung nach Anspruch 2, umfassend weiterhin in die Tropen der Komponente (b) eingebrachtes, wasserunlösliches Parfum, wobei das Silicon-Erweichungsmittel eine Viskosität bei 25°C von 50 centistokes oder weniger aufweist.

**Revendications**

1. Composition d'hydrogel topique, translucide, de soin de la peau comprenant:

   a) de 0,1% à 10% en poids d'un agent gélifiant polymère formant un hydrogel, calculé sur une base pondérale du polymère sec, ledit agent gélifiant étant un polymère réticulé ayant une capacité d'absorption d'au moins environ 40 grammes d'eau par gramme d'agent gélifiant et ne possédant pas une Tg inférieure à 140°C;
   b) de 0,1% à 15%, de préférence de 0,2% à 5%, en poids d'un émollient de type silicone insoluble dans l'eau, ledit émollient étant sous forme de gouttelettes ayant une granulométrie moyenne en nombre de 5 microns à 4000 microns, de préférence, de 10 microns à 1000 microns, plus particulièrement, de 25 microns à 500 microns; et
   c) de 50% à 99,8%, de préférence de 65% à 99,3%, en poids d'eau; dans laquelle ladite composition est limpide et possède une viscosité à 25°C d'au moins 4000 centipoises et est essentiellement dépourvue de tensioactifs.

2. Composition de soin de la peau selon la revendication 1, caractérisée en ce que ledit agent gélifiant polymère formant un hydrogel est un polymère réticulé ayant une capacité d'absorption d'au moins environ 60 grammes d'eau par gramme d'agent gélifiant et ne possède pas une Tg inférieure à 180°C, et de préférence, ne possède pas une Tg avant la décomposition dudit agent gélifiant polymère à une température de 300°C ou plus.

3. Composition de soin de la peau selon la revendication 2, caractérisée en ce que ledit agent gélifiant polymère formant un hydrogel est un polymère réticulé ayant une capacité d'absorption d'au moins environ 80 grammes d'eau par gramme d'agent gélifiant et ne possède pas une Tg inférieure à 180°C, et de préférence, ne possède pas une Tg avant la décomposition dudit agent gélifiant polymère à une température de 300°C ou plus.

4. Composition de soin de la peau selon la revendication 2, caractérisée en ce que ledit agent gélifiant polymère formant un hydrogel est un polymère réticulé comprenant des monomères d'acide carboxylique à insaturation monoéthylénique, ou des anhydrides ou des sels de ceux-ci.

5. Composition de soin de la peau selon la revendication 2, caractérisée en ce que ledit agent gélifiant polymère formant un hydrogel est choisi dans le groupe constitué par le polymère d'acide polyacrylique, le polymère d'acide polyméthacrylique, le copolymère d'acide polyacrylique/acide polyméthacrylique, les polymères dérivés d'anhydrides et de sels d'acide acrylique et d'acide méthacrylique et leurs mélanges.

**6.** Composition de soin de la peau selon la revendication 2, caractérisée en ce que ladite composition comprend de 0,5% à 5%, de préférence de 0,2% à 5%, en poids dudit émollient qui est choisi dans le groupe constitué par les silicones volatiles, les silicones non volatiles, et leurs mélanges, et de 65% à 99,3%, de préférence d'environ 65% à 99,2%, en poids d'eau.

**7.** Composition de soin de la peau selon la revendication 2, caractérisée en ce que ladite composition comprend:

a) de 0,3% à 1% en poids dudit agent gélifiant polymère formant un hydrogel;
b) de 0,5% à 2% en poids dudit émollient de type silicone, ledit émollient étant présent sous forme de gouttelettes ayant une granulométrie moyenne en nombre de 40 microns à 250 microns; et
c) de 80% à 99,2% en poids d'eau;

ladite composition ayant une viscosité à 25°C de 7000 centipoises à 35 000 centipoises.

**8.** Composition de soin de la peau selon la revendication 2, comprenant en outre un parfum insoluble dans l'eau, incorporé dans lesdites gouttelettes de constituant (b), dans laquelle ledit émollient de type silicone possède une viscosité à 25°C de 50 centistokes ou moins.